(19) Europäisches Patentamt
European Patent Office
Office européen des brevets

(11) Veröffentlichungsnummer: **0 085 653**
**B1**

(12) # EUROPÄISCHE PATENTSCHRIFT

(45) Veröffentlichungstag der Patentschrift: **11.09.85**

(51) Int. Cl.⁴: **C 07 C 87/24**, C 07 C 87/29, C 07 C 87/452 // C07C121/30, C07C121/70, C08G69/26

(21) Anmeldenummer: **83810029.5**

(22) Anmeldetag: **24.01.83**

(54) **1,10-Substituierte 1,11-Diamino-undeca-3,7-diene und Verfahren zu deren Herstellung.**

(30) Priorität: **29.01.82 CH 560/82**

(43) Veröffentlichungstag der Anmeldung: **10.08.83 Patentblatt 83/32**

(45) Bekanntmachung des Hinweises auf die Patenterteilung: **11.09.85 Patentblatt 85/37**

(84) Benannte Vertragsstaaten: **CH DE FR GB IT LI NL**

(56) Entgegenhaltungen:
**EP - A - 0 010 520**
**EP - A - 0 011 599**

(73) Patentinhaber: **CIBA-GEIGY AG, Postfach, CH-4002 Basel (CH)**

(72) Erfinder: **Reinehr, Dieter, Dr., Wolfsheule 10, D-7842 Kandern (DE)**
Erfinder: **Pfeifer, Josef, Dr., Brunnmattstrasse 32, CH-4106 Therwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Beschreibung

Die Erfindung betrifft neue 1,10-substituierte 1,11-Diamino-undeca-3,7-diene und Verfahren zu deren Herstellung. Die neuen 1,11-Diamino-undeca-3,7-diene sind wertvolle Zwischenprodukte, vor allem als Polykondensationskomponenten zur Herstellung von vernetzbaren Polyamiden.

Die neuen 1,11-Diamino-undeca-3,7-diene entsprechen der Formel I

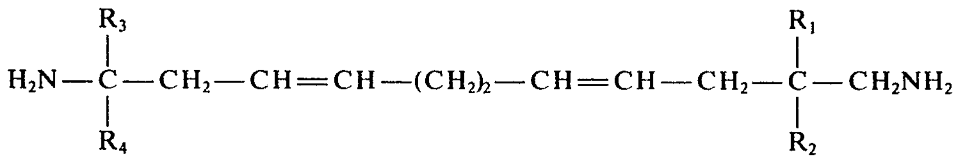

(I)

worin

$R_1$ Alkyl mit 1—12 C-Atomen,
$R_2$ Wasserstoff oder Alkyl mit 1—12 C-Atomen,
$R_3$ Alkyl mit 1—12 C-Atomen, gegebenenfalls durch $C_{1-4}$-Alkylgruppen substituiertes Cycloalkyl mit 4—12 Ring C-Atomen, Aralkyl mit 7 oder 8 C-Atomen, gegebenenfalls substituiertes Aryl, oder, wenn $R_4$ Wasserstoff ist,
auch —CH=CH-Alkyl oder —C(Alkyl)=CH-Alkyl mit je 1—4 C-Atomen in den Alkylgruppen und
$R_4$ Wasserstoff, Alkyl mit 1—12 C-Atomen, gegebenenfalls durch $C_{1-4}$-Alkylgruppen substituiertes Cycloalkyl mit 4—12 Ring-C-Atomen, Aralkyl mit 7 oder 8 C-Atomen oder gegebenenfalls substituiertes Aryl darstellen oder
$R_1$ und $R_2$ und/oder $R_3$ und $R_4$ zusammen Alkylen mit 3—11 C-Atomen bedeuten.

Durch $R_1$ bis $R_4$ dargestellte Alkylgruppen können geradkettig oder verzweigt sein. Alkylgruppen $R_1$, $R_2$ und $R_4$ weisen bevorzugt 1—5 C-Atome auf und sind geradkettig. Alkylgruppen $_3$ weisen mit Vorteil 1—7 C-Atome und besonders 1—5 C-Atome auf. Beispiele von Alkylgruppen $R_1$ bis $R_4$ sind: die Methyl-, Äthyl-, n-Propyl-, Isopropyl-, n-, sek.- und tert.-Butyl-, n-Pentyl-, 2- oder 3-Pentyl-, n-Hexyl-, 2- oder 3-Heptyl-, n-Octyl-, n-Decyl- und n-Dodecylgruppe.

Stellt $R_3$ eine Gruppe —CH=CH-Alkyl oder —C(Alkyl)=CH-Alkyl dar, so sind die Alkylgruppen in diesen Substituenten bevorzugt geradkettig und bedeuten insbesondere Methyl oder Äthyl.

Cycloalkylgruppen $R_3$ und $R_4$ können unsubstituiert oder durch $C_1$—$C_4$-Alkylgruppen substituiert sein. Insbesondere handelt es sich dabei um durch eine Methyl- oder Äthylgruppe substituiertes Cycloalkyl. Bevorzugt sind Cycloalkylgruppen $R_3$ und $R_4$ jedoch unsubstituiert und weisen 5—8 Ring-C-Atome auf. Besonders bevorzugt sind die Cyclopentyl- und vor allem die Cyclohexylgruppe.

Als Aralkylgruppen $R_3$ und $R_4$ kommen vor allem die Benzyl-, Methylbenzyl- oder Phenyläthylgruppe in Betracht. Stellt $R_3$ oder $R_4$ substituiertes Aryl dar, so kommen als Substituenten vor allem Alkylgruppen mit 1—4 und insbesondere 1 oder 2 C-Atomen in Betracht. Arylgruppen $R_3$ und $R_4$ können mehrere Alkylgruppen tragen, sind aber bevorzugt nur durch eine Alkylgruppe substituiert. Besonders bevorzugt sind die 1- oder 2-Naphthylgruppe, durch eine Alkylgruppe mit 1—4 und besonders 1 oder 2 C-Atomen substituiertes Phenyl und ganz besonders unsubstituiertes Phenyl.

Durch $R_1$ und $R_2$ und/oder $R_3$ und $R_4$ zusammen dargestellte Alkylengruppen weisen bevorzugt 4—7 C-Atome auf. Insbesonders handelt es sich dabei um die Tetramethylen- und ganz besonders die Pentamethylengruppe.

Bevorzugt sind Verbindungen der Formel I, worin $R_1$ Alkyl mit 1—5 C-Atomen, $R_2$ Wasserstoff oder Alkyl mit 1—5 C-Atomen oder $R_1$ und $R_2$ zusammen Alkylen mit 4—7 C-Atomen, $R_3$ Alkyl mit 1—7 C-Atomen, Cycloalkyl mit 5—8 C-Atomen oder unsubstituiertes Phenyl oder, bei $R_4 = H$, auch $—C(C_2H_5)=CHCH_3$ und $R_4$ Wasserstoff oder Alkyl mit 1—5 C-Atomen bedeuten. Besonders bevorzugt sind Verbindungen der Formel I, worin $R_1$ Alkyl mit 1—5 C-Atomen, $R_2$ Wasserstoff oder Alkyl mit 1—5 C-Atomen oder $R_1$ und $R_2$ zusammen Alkylen mit 4—7 C-Atomen, $R_3$ Alkyl mit 1—5 C-Atomen, unsubstituiertes Phenyl oder, bei $R_4 = H$, $—C(C_2H_5)=CHCH_3$ und $R_4$ Wasserstoff oder Methyl bedeuten, vor allem Verbindungen der Formel I, worin $R_1$ Methyl oder Äthyl, $R_2$ Wasserstoff, Methyl oder Äthyl, $R_3$ Alkyl mit 1—5 C-Atomen oder unsubstituiertes Phenyl und $R_4$ Wasserstoff oder Methyl bedeuten. Ganz besonders bevorzugt ist die Verbindung der Formel I, worin $R_1$ und $R_2$ Methyl, $R_3$ Isopropyl und $R_4$ Wasserstoff darstellen.

Als Beispiele spezifischer Verbindungen der Formel I seien genannt:

2,2-Dimethyl-11-methyl-1,11-diamino-undeca-4,8-dien,
2,2-Dimethyl-11-äthyl-1,11-diamino-undeca-4,8-dien,
2,2-Dimethyl-11-isopropyl-1,11-diamino-undeca-4,8-dien,
2,2-Dimethyl-11-n-hexyl-1,11-diamino-undeca-4,8-dien,
2,2-Dimethyl-11-phenyl-1,11-diamino-undeca-4,8-dien,
2,2-Dimethyl-11-cyclohexyl-1,11-diamino-undeca-4,8-dien,

2,2-Diäthyl-11-isopropyl-1,11-diamino-undeca-4,8-dien,
2,2-Diäthyl-11-(3-pentyl)-1,11-diamino-undeca-4,8-dien,
2,2-Dimethyl-11,11-pentamethylen-1,11-diamino-undeca-4,8-dien,
2,2-Diäthyl-11-phenyl-1,11-diamino-undeca-4,8-dien,
2-Methyl-11-phenyl-1,11-diamino-undeca-4,8-dien,
2-Methyl-2-n-propyl-11-(2-pentyl)-1,11-diamino-undeca-4,8-dien,
2-Methyl-11-cyclohexyl-1,11-diamino-undeca-4,8-dien,
2-Methyl-11-(3-pentyl)-1,11-diamino-undeca-4,8-dien,
2-Äthyl-2-n-butyl-11-(3-heptyl)-1,11-diamino-undeca-4,8-dien,
2-n-Butyl-2,11-diäthyl-1,11-diamino-undeca-4,8-dien,
2-Äthyl-2-n-butyl-11-phenyl-1,11-diamino-undeca-4,8-dien,
2,11,11-Trimethyl-1,11-diamino-undeca-4,8-dien,
2,2-Pentamethylen-11-cyclohexyl-1,11-diamino-undeca-4,8-dien,
2,2-Tetramethylen-11-cyclopentyl-1,11-diamino-undeca-4,8-dien,
2,2,11,11-Tetramethyl-1,11-diamino-undeca-4,8-dien.

Die Verbindungen der Formel I können z. B. dadurch erhalten werden, daß man Verbindungen der Formel II

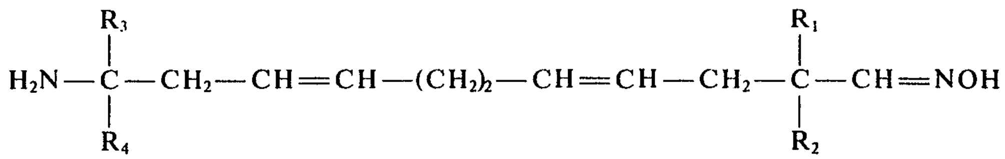

(II)

entweder direkt zu Verbindungen der Formel I reduziert oder die Verbindungen der Formel II zuerst zu Verbindungen der Formel III

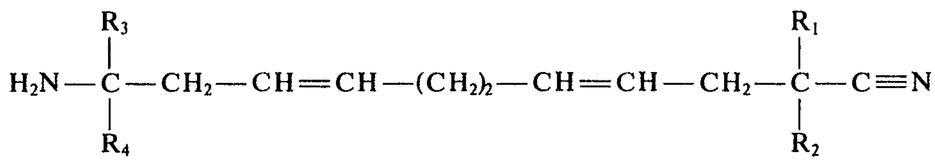

(III)

dehydratisiert und anschließend die Verbindungen der Formel III zu Verbindungen der Formel I reduziert, wobei $R_1$ und $R_4$ die unter Formel I angegebene Bedeutung haben.

Die Reduktion der Verbindungen der Formel II oder III zu Verbindungen der Formel I kann in an sich bekannter Weise erfolgen, z. B. mittels Bouveault-Blanc-Reduktion mit Natriummetall und Alkoholen, gegebenenfalls in Gegenwart eines inerten organischen Lösungsmittels, z. B. aromatischen Kohlenwasserstoffen, wie Benzol, Toluol oder Xylol. Bevorzugtes Lösungsmittel ist Toluol.

Als Alkohole verwendet man dabei zweckmäßig Alkanole mit bis zu 6 C-Atomen, wie Methanol, Äthanol, Propanol, Isopropanol, Butanole und Hexanole. Bevorzugt ist Isopropanol. Die Reaktionstemperaturen für die Reduktion mit Natriummetall und Alkoholen liegen zweckmäßig zwischen 25°C und der Rückflußtemperatur des Reaktionsgemisches, bevorzugt zwischen 60 und 120°C.

Die Reduktion kann auch mit Lithiumaluminiumhydrid, gegebenenfalls zusammen mit Aluminiumchlorid in wasserfreiem, bevorzugt organischem Medium, z. B. Diäthyläther, Tetrahydrofuran, Benzol, Toluol oder n-Hexan, zweckmäßig bei Temperaturen zwischen 0 und 80°C vorgenommen werden. Weitere geeignete Reduktionsmittel sind Natriumboranat, Zinkstaub oder Zink in Eisessig oder Natriumamalgam in saurem Medium. Die beiden letztgenannten Reduktionsmittel eignen sich besonders für die direkte Reduktion der Oxime der Formel II. Bevorzugt ist die Reduktion mit Natriummetall und Alkoholen in Gegenwart eines inerten organischen Lösungsmittels, wobei in bezug auf den Alkohol, das Lösungsmittel und die Reaktionstemperaturen die oben angegebenen Bevorzugungen gelten.

Die allfällige Dehydratisierung der Verbindungen der Formel II zu Verbindungen der Formel III kann ebenfalls in an sich bekannter Weise chemisch oder thermisch durchgeführt werden.

Als Dehydratisierungsmittel für die chemische Dehydratisierung eignen sich z. B. Anhydride von gegebenenfalls durch Halogenatome oder Alkylgruppen substituierten aliphatischen Monocarbonsäuren mit 2—5 C-Atomen, wie Essigsäure-, Propionsäure-, Buttersäure- und Valeriansäureanhydrid, Trichlor-, Trifluor-, Trimethyl-, Triäthyl- und Tri-n-butylessigsäureanhydrid; Alkylester mit 1—5 C-Atomen der Chlorameisensäure, wie der Chlorameisensäuremethyl-, -äthyl-, -isopropyl- und -isobutylester; Acetylchlorid, Thionylchlorid, Benzoylchlorid, Benzolsulfonylchlorid, Sulfurylchlorid, Phosphorpentoxid im Gemisch mit Äthanol, Phosphoroxichlorid, Polyphosphorsäure und wäßriges Alkali, wie wäßriges NaOH oder KOH. Die genannten Dehydratisierungsmittel können gegebenenfalls im Gemisch mit Katalysatoren, z. B. Erdalkalimetall- oder Alkalimetallsalzen von aromatischen Monocarbonsäuren oder von aliphatischen Monocarbonsäuren mit 1—3 C-Atomen, wie Natriumbenzoat, Natriumsalicylat, Calcium- und Natriumformiat, Calcium-, Magnesium, Natrium- und Kaliumacetat und Natriumpropionat

oder tertiären Basen, wie Triäthylamin, Pyridin und Dimethylanilin, eingesetzt werden.

Bevorzugtes Dehydratisierungsmittel ist Essigsäureanhydrid. Die chemische Dehydratisierung wird mit Vorteil in Gegenwart eines unter den Reaktionsbedingungen inerten organischen Verdünnungs- oder Lösungsmittels durchgeführt. Geeignete Verdünnungs- bzw. Lösungsmittel sind z. B. aromatische Kohlenwasserstoffe, wie Benzol und Toluol, aromatische oder aliphatische Nitrile, besonders Benzonitril und Alkylnitrile mit 2–5 C-Atomen, wie Acetonitril, Propionitril und Butyronitril, sowie wasserfreie Essigsäure. Bevorzugte Verdünnungs- bzw. Lösungsmittel sind Acetonitril und vor allem wasserfreie Essigsäure.

Die chemische Dehydratisierung wird im allgemeinen bei Temperaturen zwischen 50°C und der Rückflußtemperatur des Reaktionsgemisches vorgenommen.

Die thermische Dehydratisierung kann z. B. über Aluminiumoxid oder Thoriumoxid bei Temperaturen zwischen 340 und 360°C durchgeführt werden. Die chemische Dehydratisierung ist bevorzugt.

Die Ausgangsprodukte der Formel II sind bekannt und können nach dem in dem europäischen Patent 11 599 beschriebenen Verfahren hergestellt werden.

Die Diamine der Formel I sind wertvolle Zwischenprodukte und finden z. B. Anwendung als Polykondensationskomponenten zur Herstellung von transparenten vernetzbaren Polyamiden. Solche Polyamide können auf an sich bekannte Weise durch Polykondensation von Diaminen der Formel I mit gesättigten oder ungesättigten aliphatischen und/oder aromatischen Dicarbonsäuren oder amidbildenden Derivaten davon, vorzugsweise in Gegenwart einer anorganischen oder organischen Phosphorverbindung als Beschleuniger, hergestellt werden. Sie können thermisch oder photochemisch vernetzt werden, bevorzugt in Gegenwart von radikalischen Initiatoren. Die photochemische Vernetzung erfolgt mit Vorteil durch Polythiole mit mindestens zwei Thiolgruppen pro Molekül in Gegenwart von Photosensibilisatoren, wie Benzophenon, Thioxanthon und dergleichen. Die genannten Polyamide bzw. deren Gemische mit Polythiolen und Sensibilisatoren eignen sich z. B. zur Herstellung von lösungsmittelbeständigen Überzügen auf verschiedenen Materialien, besonders Metallen, zur Bilderzeugung unter Lichteinwirkung oder zur Herstellung von transparenten Formkörpern, z. B. nach dem Spritzguß- oder Extrusionsverfahren. Die unter Verwendung von Diaminen der Formel I und aromatischen und/oder aliphatischen Dicarbonsäuren, besonders Terephthalsäure, Isophthalsäure und/oder Adipinsäure, erhaltenen transparenten Polyamide zeichnen sich durch geringe Wasseraufnahme, hohe Hydrolysebeständigkeit und/oder gute Dimensionsstabilität unter Feuchtigkeitseinwirkung aus. Sie ergeben ferner sehr gut haftende, lösungsmittelbeständige Überzüge und weisen im Gegensatz zu vorbekannten ungesättigten Polyamiden eine gute Verträglichkeit mit Polythiolen auf. Dank ihrer guten Löslichkeit in verschiedenen organischen Lösungsmitteln lassen sie sich auch leicht thermisch vernetzen.

## A) Herstellungsbeispiele

### Beispiel 1

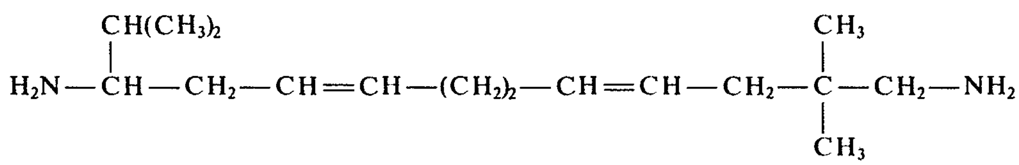

a) 80 g (0,3 Mol) 2,2-Dimethyl-11-isopropyl-1,11-amino-undeca-4,8-dienaloxim werden unter Rühren mit 100 ml Eisessig versetzt. Dann wird HCl-Gas bis zur Sättigung eingeleitet, und innerhalb von 15 Minuten werden 30,6 g (0,3 Mol) Essigsäureanhydrid zugetropft. Man erhitzt die Reaktionsmischung noch 4 Stunden unter Rückfluß, destilliert den Eisessig ab und löst den Rückstand in Wasser. Nachdem man die Lösung mit Natronlauge basisch gestellt hat, nimmt man die sich abscheidende organische Phase mit Toluol auf und destilliert. Man erhält 68,5 g (0,276 Mol) 1,1-Dimethyl-10-isopropyl-10-amino-deca-3,7-dien-nitril, entsprechend einer Ausbeute von 92% d. Th.; Sdp. 94°C/3 Pa.
$^1$H-NMR-Spektrum τ (ppm): 4,5 (m), 7,45 (quin), 7,9 (m), 2,0–2,5 (m), 8,65 (s), 8,84 (s), 9,07 (dd) im Verhältnis 4 : 1 : 8 : 1 : 6 : 2 : 6.

b) 23 g (1 Mol) Natrium werden zu 150 ml Toluol gegeben, und die Mischung wird bis zum Schmelzen des Natriums aufgeheizt. Dann wird die Heizung entfernt und so lange gerührt, bis das Natrium als feingraue Dispersion zerteilt ist. Zu dieser Mischung tropft man dann eine Lösung von 53 g (0,214 Mol) 1,1-Dimethyl-10-isopropyl-10-amino-deca-3,7-dien-nitril in 100 ml Isopropanol. Es wird noch 3 Stunden unter Rückfluß gekocht, mit 200 ml Wasser versetzt, und die organische Phase wird abgetrennt. Nach dem Abdestillieren des Lösungsmittels erhält man 44 g (0,175 Mol) 2,2-Dimethyl-11-isopropyl-1,11-diamino-undeca-4,8-dien, entsprechend einer Ausbeute von 81,5% d. Th.; Sdp. 86°C/1 Pa; $n_D^{20} = 1,4810$.

Analyse für $C_{16}H_{32}N_2$ (Molgewicht 252,45):

| | | | |
|---|---|---|---|
| berechnet | C 76,12% | H 12,78% | N 11,10%; |
| gefunden | C 75,59% | H 12,97% | N 10,82%. |

$^1$H-NMR-Spektrum τ (ppm): 4,6 (m), 7,47 (m) u. 7,58 (s), 7,7 bis 8,5 (m), 8,96 (s), 9,07 (dd) und 9,13 (s) im Verhältnis von 4 : 3 : 9 : 4 : 12.
MS-Spektrum: Molekülpeak 252, Bruchstückmassen 237, 201, 192, 181, 126, 72.

## Beispiel 2

a) Es wird wie in Beispiel 1a) beschrieben vorgegangen, jedoch unter Verwendung von 50,5 g (0,2 Mol) 2,2-Dimethyl-11-äthyl-11-amino-undeca-4,8-dienal-oxim, 25 g (0,245 Mol) Essigsäureanhydrid und 100 ml Acetonitril als Lösungsmittel. Nach der Aufarbeitung erhält man 37 g (0,159 Mol) 1,1-Dimethyl-10-äthyl-10-amino-deca-3,7-diennitril, entsprechend einer Ausbeute von 79,5% d. Th.; Sdp. 84°C/1 Pa; $n_D^{20}$ = 1,4711.
$^1$H-NMR-Spektrum τ (ppm): 4,5 (m), 7,3 (m), 7,8 (m), 8,3—8,75 (m), 8,82 (s) 9,03 (t) im Verhältnis von 4 : 1 : 8 : 8 : 2 : 3.
MS-Spektrum: Molekülpeak 234, Bruchstückmassen 205, 166, 82.

b) Es wird wie in Beispiel 1b) beschrieben vorgegangen, jedoch unter Verwendung von 30 g (0,128 Mol) 1,1-Dimethyl-10-äthyl-10-amino-deca-3,7-dien-nitril, 100 ml Isopropanol, 23 g (1 Mol) Natrium und 100 ml Xylol. Nach der Aufarbeitung erhält man 22 g (0,925 g) 2,2-Dimethyl-11-äthyl-1,11-diamino-undeca-4,8-dien, entsprechend einer Ausbeute von 71,8% d. Th.; Sdp. 80°C/1 Pa; $n_D^{20}$ = 1,4809.

Analyse für $C_{15}H_{30}N_2$ (Molgewicht 238,42):

| | | | |
|---|---|---|---|
| berechnet | C 75,57% | H 12,69% | N 11,75%; |
| gefunden | C 75,7% | H 12,5% | N 10,7%. |

$^1$H-NMR-Spektrum τ (ppm): 4,6 (m), 7,3 (m), 7,56 (s), 7,7 bis 8,2 (m), 8,6 (m) und 8,79 (s), 9,03 (t) und 9,12 (s) im Verhältnis von 4 : 1 : 2 : 8 : 6 : 9.
MS-Spektrum: Molekülpeak 238, Bruchstückmassen 223, 209, 181, 166, 126, 98.

## Beispiel 3

a) Es wird wie in Beispiel 1a) beschrieben vorgegangen, jedich unter Verwendung von 473 g (1,65 Mol) 2-Methyl-11-phenyl-11-amino-undeca-4,8-dienal-oxim, 169 g (1,65 Mol) Essigsäureanhydrid, 250 ml Essigsäure und HCl-Gas im Überschuß. Nach der Aufarbeitung erhält man 160 g (0,6 Mol) 1-Methyl-10-phenyl-10-amino-deca-3,7-dien-nitril, entsprechend einer Ausbeute von 36,4% d. Th.; Sdp. 135°C/1 Pa; $n_D^{20}$ = 1,5268.
$^1$H-NMR-Spektrum τ (ppm): 2,72 (s), 4,55 (m), 6,09 (t), 7,4 (m), 7,6 bis 8,0 (m), 8,33 (s), 8,72 (d) im Verhältnis von 5 : 4 : 1 : 1 : 8 : 2 : 3.
MS-Spektrum: Molekülpeak 268, Bruchstückmassen 214, 146, 106, 79.

b) Es wird wie im Beispiel 1b) beschrieben vorgegangen, jedoch unter Verwendung von 134 g (0,5 Mol) 1-Methyl-10-phenyl-10-amino-deca-3,7-dien-nitril, 58,5 g (2,54 Mol) Natrium, 500 ml Toluol und 250 ml Isopropanol. Nach der Aufarbeitung erhält man 60,5 g (0,223 Mol) 2-Methyl-11-phenyl-1,11-diamino-undeca-4,8-dien, entsprechend einer Ausbeute von 44,5% d. Th.; Sdp. 103°C/13 Pa; $n_D^{20}$ = 1,5293.

Analyse für $C_{18}H_{28}N_2$ (Molgewicht 272,44):

| | | | |
|---|---|---|---|
| berechnet | C 79,36% | H 10,36% | N 10,28%; |
| gefunden | C 79,24% | H 10,55% | N 10,02%. |

$^1$H-NMR-Spektrum τ (ppm): 2,7 (m), 4,6 (m), 6,09 (t), 7,2—8,2 (m), 8,5 (m), 8,70 (s), 9,08 (d) im Verhältnis von 5 : 4 : 1 : 10 : 1 : 4 : 3.
MS-Spektrum: Molekülpeak 272, Bruchstückmassen 254, 167, 106, 79.

## Beispiel 4

a) Es wird wie in Beispiel 1a) beschrieben vorgegangen, jedoch unter Verwendung von 148 g (0,62 Mol) 2,11,11-Trimethyl-11-amino-undeca-4,8-dienal-oxim, 63,3 g (0,62 Mol) Acetanhydrid, 250 ml Eisessig und HCl-Gas im Überschuß. Nach der Aufarbeitung erhält man 87 g (0,396 Mol) 1,10,10-Trimethyl-10-amino-deca-3,7-dien-nitril, entsprechend einer Ausbeute von 63,7% d. Th.; Sdp. 94°C/4 Pa; $n_D^{20}$ = 1,4706.

$^1$H-NMR-Spektrum $\tau$ (ppm): 4,5 (m), 7,35 (sex), 7,6–8,1 (m), 8,38 (s), 8,68 (d), 8,88 (s) im Verhältnis von 4 : 1 : 8 : 2 : 3 : 6.
MS-Spektrum: Molekülpeak 220, Bruchstückmassen 205, 190, 178, 98, 58.

b) Es wird wie in Beispiel 1b) beschrieben vorgegangen, jedoch unter Verwendung von 83 g (0,378 Mol) 1,10,10-Trimethyl-10-amino-deca-3,7-dien-nitril, 60 g (2,6 Mol) Natrium, 500 ml Toluol und 250 ml Isopropanol. Nach der Aufarbeitung erhält man 68 g (0,304 Mol) 2,11,11-Trimethyl-1,11-diamino-undeca-4,8-dien, entsprechend einer Ausbeute von 80,4% d. Th.; Sdp. 80°C/2 Pa; $n_D^{20}$ = 1,4785.

Analyse für $C_{14}H_{28}N_2$ (Molgewicht 224,39):

| | | | |
|---|---|---|---|
| berechnet | C 74,94% | H 12,58% | N 12,49%; |
| gefunden | C 74,72% | H 12,84% | N 12,34%. |

$^1$H-NMR-Spektrum $\tau$ (ppm): 4,6 (m), 7,42 (m), 7,9 (m), 8,5 (m), 8,88 (s), 9,07 (d) im Verhältnis von 4 : 2 : 8 : 1 : 10 : 3.
MS-Spektrum: Molekülpeak 224, Bruchstückmassen 209, 192, 167, 112, 96, 58.

## Beispiel 5

a) Es wird wie in Beispiel 1a) beschrieben vorgegangen, jedoch unter Verwendung von 150 g (0,59 Mol) 2,2,11,11-Tetramethyl-11-amino-undeca-4,8-dienal-oxim, 75 g (0,735 Mol) Acetanhydrid und 250 ml Eisessig. Nach der Aufarbeitung erhält man 135 g (0,576 Mol) 1,1,10,10-Tetramethyl-10-amino-deca-3,7-dien-nitril, entsprechend einer Ausbeute von 97,6% d. Th.; Sdp. 85°C/1 Pa.

b) Es wird wie in Beispiel 1b) beschrieben vorgegangen, jedoch unter Verwendung von 50 g (0,213 Mol) 1,1,10,10-Tetramethyl-10-amino-deca-3,7-dien-nitril, 95 g (4,13 Mol) Natrium, 500 ml Toluol und 300 ml Isopropanol. Nach der Aufarbeitung erhält man 28,5 g (0,12 Mol) 2,2,11,11-Tetramethyl-1,11-diamino-undeca-4,8-dien, entsprechend einer Ausbeute von 56,3% d. Th.; Sdp. 100°C/4 Pa; $n_D^{20}$ = 1,4772.

Analyse für $C_{15}H_{30}N_2$ (Molgewicht 238,42):

| | | | |
|---|---|---|---|
| berechnet | C 75,57% | H 12,69% | N 11,75%; |
| gefunden | C 75,35% | H 12,47% | N 11,37%. |

$^1$H-NMR-Spektrum $\tau$ (ppm): 4,6 (m), 7,56 (s), 7,8 bis 8,2 (m), 8,89 (s), 9,12 (s) im Verhältnis von 4 : 2 : 8 : 10 : 6.
MS-Spektrum: Molekülpeak 238, Bruchstückmassen 223, 206, 181, 126, 81, 58.

## Beispiel 6

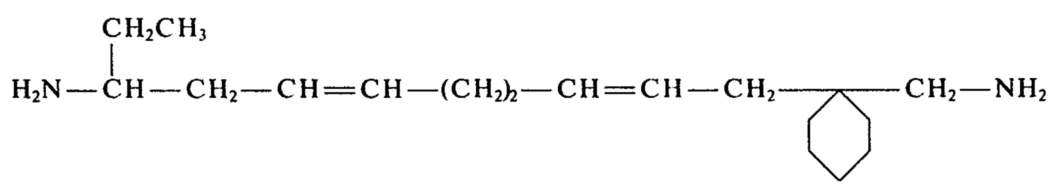

a) 332 g (1,13 Mol) 2,2-Pentamethylen-11-äthyl-11-amino-undeca-4,8-dienal-oxim werden unter Rühren mit 153 g (1,5 Mol) Acetanhydrid, 90 g Eisessig und HCl-Gas im Überschuß versetzt. Man erhitzt die Reaktionsmischung noch 4 Stunden unter Rückfluß, destilliert den Eisessig ab und löst den Rückstand in Wasser. Nachdem man die Lösung mit Natronlauge basisch gestellt hat, nimmt man die sich abscheidende organische Phase mit Toluol auf und destilliert. Man erhält 229 g (0,833 Mol 1,1-Pentamethylen-10-äthyl-10-amino-deca-3,7-dien-nitril, entsprechend einer Ausbeute von 73,8% d. Th.; Sdp. 138°C/1 Pa; $n_D^{20}$ = 1,4931.

Analyse für $C_{18}H_{30}N_2$ (Molgewicht 274,45):

| | | | |
|---|---|---|---|
| berechnet | C 78,77% | H 11,02% | N 10,21%; |
| gefunden | C 78,67% | H 10,83% | N 9,98%. |

$^1$H-NMR-Spektrum $\tau$ (ppm): 4,6 (m), 7,4 (m), 7,8–8,9 (m), 9,1 (t) im Verhältnis 4 : 1 : 22 : 3.
MS-Spektrum: Molekülpeak 274, Bruchstückmassen 259, 245, 191, 166, 112, 98.

b) Es wird wie in Beispiel 1b) beschrieben vorgegangen, jedoch unter Verwendung von 65 g (0,237 Mol) 1-Pentamethylen-10-äthyl-10-amino-deca-3,7-dien-nitril, 48 g (2,08 Mol) Natrium, 500 ml Toluol und 250 ml Isopropanol. Nach der Aufarbeitung erhält man 43 g (0,153 Mol) 2-Pentamethylen-11-äthyl-1,11-diamino-undeca-4,8-dien, entsprechend einer Ausbeute von 64,5% d. Th.; Sdp. 140°C/3 Pa.

Analyse für $C_{18}H_{34}N_2$ (Molgewicht 278,48):
berechnet C 77,63% H 12,31% N 10,06%;
gefunden C 78,20% H 12,29% N 9,73%.

$^1$H-NMR-Spektrum τ (ppm): 4,6 (m), 7,35 (m) und 7,48 (s), 7,9 (m), 8,5 (m), 8,92 (s), 9,04 (t) im Verhältnis von 4 : 3 : 8 : 12 : 4 : 3.
MS-Spektrum: Molekülpeak 278, Bruchstückmassen 249, 221, 166, 152, 138, 112.

Beispiel 7

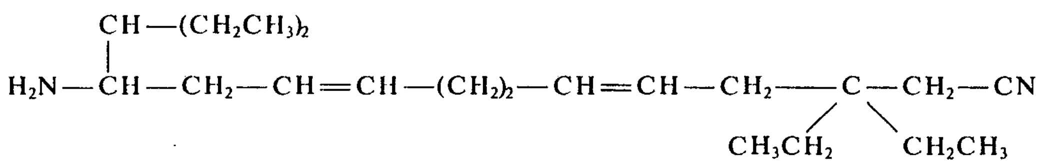

a) Es wird wie in Beispiel 6a) beschrieben vorgegangen, jedoch unter Verwendung von 280 g (0,87 Mol) 2,2-Diäthyl-11-(3-pentyl)-11-amino-undeca-4,8-dienal-oxim, 112 g (1,2 Mol) Acetanhydrid, 75 g Eisessig und HCl-Gas im Überschuß. Nach der Aufarbeitung erhält man 138 g (0,455 Mol) 1,1-Diäthyl-10-(3-pentyl)-10-amino-deca-3,7-dien-nitril, entsprechend einer Ausbeute von 52,3% d. Th.; Sdp. 139°C/3 Pa; $n_D^{20}$ = 1,4793.

Analyse für $C_{20}H_{36}N_2$ (Molgewicht 304,52):
berechnet C 78,88% H 11,92% N 9,20%;
gefunden C 78,90% H 11,63% N 9,45%.

$^1$H-NMR-Spektrum τ (ppm): 4,6 (m), 7,3 (m), 7,7–8,2 (m), 8,4–9,2 (m) im Verhältnis von 4 : 1 : 8 : 23.
MS-Spektrum: Molekülpeak 303, Bruchstückmassen 275, 233, 142, 100, 82.

b) Es wird wie in Beispiel 1b) beschrieben vorgegangen, jedoch unter Verwendung von 69,8 g (0,23 Mol) 1,1-Diäthyl-10-(3-pentyl)-10-amino-deca-3,7-dien-nitril, 35 g (1,52 Mol) Natrium, 250 ml Toluol und 70 ml Isopropanol. Nach der Aufarbeitung erhält man 50 g (0,162 Mol) 2,2-Diäthyl-11-(3-pentyl)-1,11-diamino-undeca-4,8-dien, entsprechend einer Ausbeute von 70,5% d. Th.; Sdp. 130°C/4 Pa; $n_D^{20}$ = 1,4861.

Analyse für $C_{20}H_{40}N_2$ (Molgewicht 308,55):
berechnet C 77,85% H 13,07% N 9,08%;
gefunden C 77,92% H 13,31% N 8,94%.

$^1$H-NMR-Spektrum τ (ppm): 4,6 (m), 7,25 (m), 7,57 (s), 7,7–8,8 (m), 9,02 (s) und 9,1 (m) im Verhältnis 4 : 1 : 2 : 17 : 16.
MS-Spektrum: Molekülpeak 308, Bruchstückmassen 279, 262, 237, 220, 209, 180, 154, 140, 126, 100.

B) Verwendungsbeispiele

Beispiel I

62,8 g 2,2-Dimethyl-11-isopropyl-1,11-diamino-undeca-4,8-dien, 36,4 g Adipinsäure, 0,25 ml einer 10%igen wäßrigen $NH_4H_2PO_2$-Lösung und 0,5 g Di-tert.-butyl-p-kresol werden in einem Autoklaven 90 Minuten in Stickstoffatmosphäre vorkondensiert, dann 4 Stunden in einem offenen Polykondensationsgefäß im Stickstoffstrom weiterkondensiert und schließlich 1 Stunde im Hochvakuum nachkondensiert. Alle Polykondensationsschritte werden bei 250°C durchgeführt.

Elementaranalyse des erhaltenen Polyamids:
berechnet C 72,88% H 10,57% N 7,73%;
gefunden C 70,20% H 10,47 N 7,45%.

Endgruppengehalt: –COOH 0,16 m Äquiv./g; $-NH_2$ 0,04 m Äquiv./g. Glasumwandlungstemperatur Tg 45°C [bestimmt im Differentialkalorimeter (DSC)]; reduzierte Viskosität (0,5%ige Lösung in m-Kresol bei 25°C) $\eta_{red}$ = 0,70 dl/g.

Beispiel II

a) Herstellung eines Salzes aus Terephthalsäure (TPS) und 2,2-Dimethyl-11-isopropyl-1,11-diamino-undeca-4,8-dien (Undeca-dien-diamin = UDD):
97,1 g Terephthalsäure werden in 2400 ml Wasser und 600 ml Methanol suspendiert. Zur erhaltenen Suspension tropft man bei Rückflußtemperatur 148,9 g UDD zu, wobei eine homogene Lösung entsteht. Nach 60 Minuten Kochen läßt man die Lösung auf 0–5°C abkühlen. Nach 24 Stunden wird vom gebildeten Salz abfiltriert und bei 80°C im Vakuum getrocknet. Ausbeute 180 g (73,6% d. Th.).

b) Herstellung eines Salzes aus Adipinsäure (AS) und UDD:
36,2 g Adipinsäure werden in 270 ml absolutem Äthanol bei 50°C gelöst. Nach dem Abkühlen der Lösung werden 69,7 g UDD in 107 ml absolutem Äthanol zugegeben. Das Salz, das nach Abziehen von zwei Dritteln des Lösungsmittels und Abkühlen des Restes auf 0–5°C ausfällt, wird abfiltriert und im Vakuum bei 20°C getrocknet. Ausbeute 82,5 g (85,3% d. Th.).
56,0 g des gemäß a) erhaltenen Salzes aus TPS und UDD sowie 14,0 g des obigen Salzes aus Adipinsäure und UDD werden wie in Beispiel I beschrieben polykondensiert, jedoch unter Weglassen der letzten Kondensationsstufe im Vakuum. Glasumwandlungtemperatur des Polyamids = 105°C, $\eta_{red}$ (0,5%ige Lösung in m-Kresol bei 25°C) = 0,72 dl/g.

Beispiel III

Eine 10%ige Lösung des gemäß Beispiel I hergestellten Polyamids in Chloroform/Äthanol (1 : 1), die 5 Gew.-% Cumolhydroperoxid enthält, wird mit Hilfe eines 50-μm-Rakels auf eine Kupfer-Leiterplatte gegossen. Die Beschichtung wird 3 Minuten bei 100°C getrocknet, wobei die Schichtdicke ca. 5 μm beträgt. Eine Nachhärtung bei 150°C während 90 Minuten in Stickstoffatmosphäre ergibt eine harte, transparente und glänzende Schicht mit guter Haftung auf dem Kupfer. Läßt man die so behandelte Platte 72 Stunden in 60°C warmem Chloroform stehen, so wird das Polyamid nicht abgelöst, und die Haftung des Polyamids reicht aus, um eine Ätzung des darunterliegenden Kupfers zu verhindern. Nach einer Woche Lagerung in Wasser bei 20°C behält die Beschichtung ihre Transparenz und gute Haftung auf dem Kupfer; Wasseraufnahme unter 1 Gew.-%.

Beispiel IV

i) 2,5 g des gemäß Beispiel I erhaltenen Polyamids, 1,5 g Pentaerythritol-tetrakis-(3-mercaptopropionat) und 95 mg Thioxanthon werden in 10,8 ml Chloroform gelöst und mit einem 50-μm-Rakel auf eine 100 μm dicke Polyesterfolie aufgetragen. Die Beschichtung wird 3 Minuten bei 100°C getrocknet und mit einem 5000 W Quecksilberhochdruckbrenner durch eine photographische Maske während 15 Sekunden belichtet (Abstand des Quecksilberhochdruckbrenners vom Vakuumtisch 70 cm). Nach 30 Sekunden Entwicklung in Chloroform erhält man ein gut aufgelöstes Negativ-Reliefbild.

ii) Das unter i) beschriebene Verfahren wird wiederholt unter Verwendung von 81,6 mg Benzophenon anstelle des Thioxanthons und einer Belichtungszeit von 60 Sekunden.

| Versuch | Belichtungszeit (Sekunden) | letzte abgebildete Stufe auf einem 21 step-sensitivity guide der Fa. Stouffer |
|---|---|---|
| i) | 15 | 8 |
| ii) | 60 | 8 |

**Patentansprüche**

1. Verbindungen der Formel I

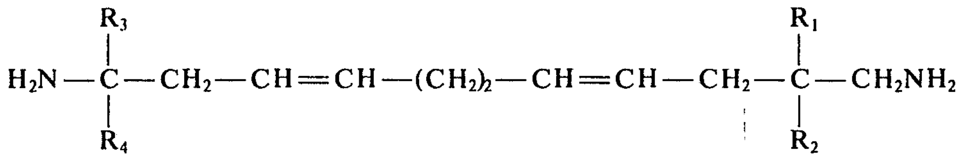

(I)

worin

$R_1$ Alkyl mit 1—12 C-Atomen,
$R_2$ Wasserstoff oder Alkyl mit 1—12 C-Atomen,
$R_3$ Alkyl mit 1—12 C-Atomen, gegebenenfalls durch $C_{1-4}$-Alkylgruppen substituiertes Cycloalkyl mit 4—12 Ring-C-Atomen, Aralkyl mit 7 oder 8 C-Atomen, gegebenenfalls substituiertes Aryl, oder, wenn $R_4$ Wasserstoff ist,
auch —CH=CH-Alkyl oder —C(Alkyl)=CH-Alkyl mit je 1—4 C-Atomen in den Alkylgruppen und
$R_4$ Wasserstoff, Alkyl mit 1—12 C-Atomen, gegebenenfalls durch $C_{1-4}$-Alkylgruppen substituiertes Cycloalkyl mit 4—12 Ring-C-Atomen, Aralkyl mit 7 oder 8 C-Atomen oder gegebenenfalls substituiertes Aryl darstellen oder
$R_1$ und $R_2$ und/oder $R_3$ und $R_4$ zusammen Alkylen mit 3—11 C-Atomen bedeuten.

2. Verbindungen der Formel I nach Anspruch 1, worin $R_1$ Alkyl mit 1—5 C-Atomen, $R_2$ Wasserstoff oder Alkyl mit 1—5 C-Atomen oder $R_1$ und $R_2$ zusammen Alkylen mit 4—7 C-Atomen, $R_3$ Alkyl mit 1—7 C-Atomen, Cycloalkyl mit 5—8 C-Atomen oder unsubstituiertes Phenyl oder, bei $R_4 = H$, auch $-C(C_2H_5)=CH-CH_3$ und $R_4$ Wasserstoff oder Alkyl mit 1—5 C-Atomen bedeuten.

3. Verbindungen der Formel I nach Anspruch 2, worin $R_3$ Alkyl mit 1—5 C-Atomen, unsubstituiertes Phenyl oder, bei $R_4 = H$, $-C(C_2H_5)=CH-CH_3$ und $R_4$ Wasserstoff oder Methyl bedeuten.

4. Verbindungen der Formel I nach Anspruch 1, worin $R_1$ Methyl oder Äthyl, $R_2$ Wasserstoff, Methyl oder Äthyl, $R_3$ Alkyl mit 1—5 C-Atomen oder unsubstituiertes Phenyl und $R_4$ Wasserstoff oder Methyl bedeuten.

5. Verbindung der Formel I nach Anspruch 1, worin $R_1$ und $R_2$ Methyl, $R_3$ Isopropyl und $R_4$ Wasserstoff bedeuten.

6. Verfahren zur Herstellung von Verbindungen der Formel I nach Anspruch 1, dadurch gekennzeichnet, daß man Verbindungen der Formel II

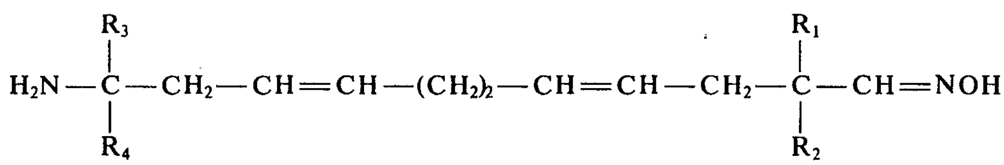

(II)

entweder direkt zu Verbindungen der Formel I reduziert oder die Verbindungen der Formel II zuerst zu Verbindungen der Formel III

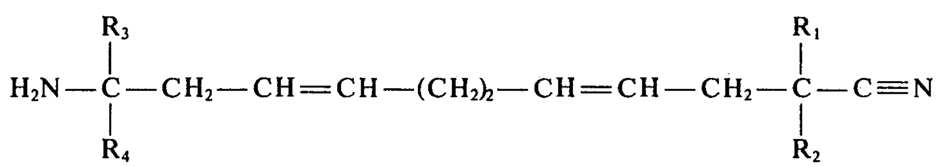

(III)

dehydratisiert und anschließend die Verbindungen der Formel III zu Verbindungen der Formel I reduziert, wobei $R_1$ bis $R_4$ die im Anspruch 1 angegebene Bedeutung haben.

# Claims

1. A compound of the formula I

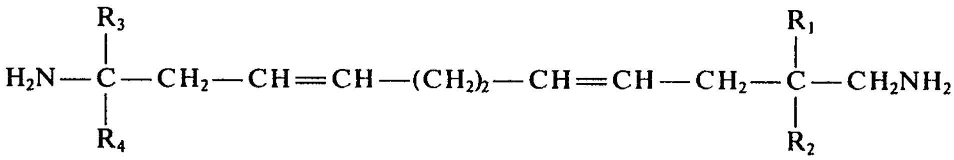

(I)

in which

$R_1$ is alkyl having 1—12 C atoms,
$R_2$ is hydrogen or alkyl having 1—12 C atoms,
$R_3$ is alkyl having 1—12 C atoms, cycloalkyl having 4—12 ring C atoms and being unsubstituted or substituted by $C_1$—$C_4$alkyl groups, aralkyl having 7 or 8 C atoms, substituted or unsubstituted aryl or, if $R_4$ is hydrogen, $R_3$ is —CH=CH-alkyl or —C(alkyl)=CH-alkyl each having 1—4 C atoms in the alkyl groups, and
$R_4$ is hydrogen, alkyl having 1—12 C atoms, cycloalkyl having 4—12 ring C atoms and being unsubstituted or substituted by $C_1$—$C_4$alkyl groups, aralkyl having 7 or 8 C atoms or substituted or unsubstituted aryl, or
$R_1$ and $R_2$ and/or $R_3$ and $R_4$ together are alkylene having 3—11 C atoms.

2. A compound of the formula I according to claim 1, in which $R_1$ is alkyl having 1—5 C atoms, $R_2$ is hydrogen or alkyl having 1—5 C atoms, or $R_1$ and $R_2$ together are alkylene having 4—7 C atoms, $R_3$ is alkyl having 1—7 C atoms, cycloalkyl having 5—8 C atoms, unsubstituted phenyl or, if $R_4 = H$, $R_3$ is $-C(C_2H_5)=CH-CH_3$, and $R_4$ is hydrogen or alkyl having 1—5 C atoms.

3. A compound of the formula I according to claim 2, in which $R_3$ is alkyl having 1—5 C atoms, unsubstituted phenyl or, if $R_4 = H$, $R_3$ is $-C(C_2H_5)=CH-CH_3$, and $R_4$ is hydrogen or methyl.

4. A compound of the formula I according to claim 1, in which $R_1$ is methyl or ethyl, $R_2$ is hydrogen, methyl or ethyl, $R_3$ is alkyl having 1—5 C atoms or unsubstituted phenyl, and $R_4$ is hydrogen or methyl.

5. A compound of the formula I according to claim 1, in which $R_1$ and $R_2$ are methyl, $R_3$ is isopropyl and $R_4$ is hydrogen.

6. A process for the preparation of a compound of the formula I according to claim 1, which comprises either reducing a compound of the formula II

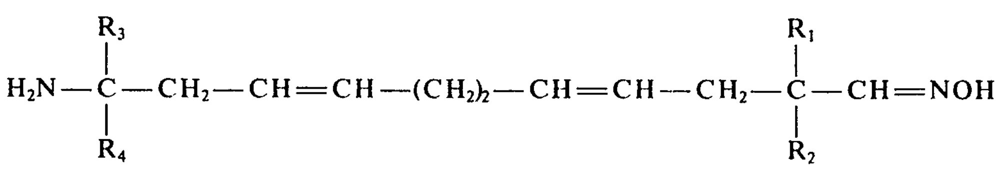

(II)

direct to give a compound of the formula I, or first dehydrating the compound of the formula II to give a compound of the formula III

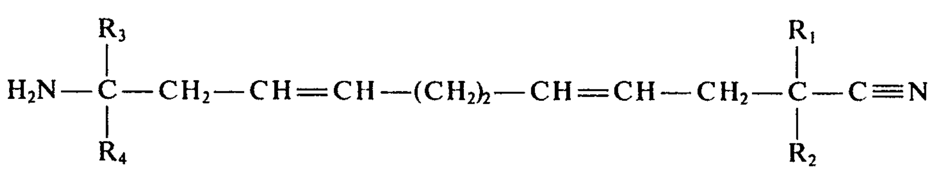

(III)

and then reducing the compound of the formula III to give a compound of the formula I, $R_1$ to $R_4$ being as defined in claim 1.

**Revendications**

1. Composés répondant à la formule I

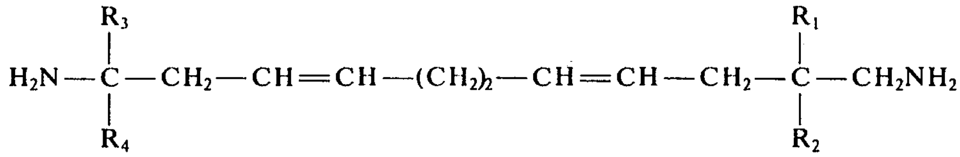

(I)

dans laquelle

$R_1$ représente un alkyle en $C_1$–$C_{12}$,
$R_2$ représente l'hydrogène ou un alkyle en $C_1$–$C_{12}$,
$R_3$ représente un alkyle en $C_1$–$C_{12}$, un cyclo-alkyle contenant de 4 à 12 atomes de carbone dans son cycle et éventuellement porteur d'alkyles en $C_1$–$C_4$, un aralkyle en $C_7$ ou $C_8$, un aryle éventuellement substitué, ou, lorsque $R_4$ représente l'hydrogène, également un radical –CH=CH-alkyl ou un radical –C(alkyl)=CH-alkyl contenant de 1 à 4 atomes de carbone dans chacun des radicaux alkyles, et
$R_4$ représente l'hydrogène, un alkyle en $C_1$–$C_{12}$, un cyclo-alkyle contenant de 4 à 12 atomes de carbone dans son cycle et éventuellement porteur d'alkyles en $C_1$–$C_4$, un aralkyle en $C_7$ ou $C_8$ ou un aryle éventuellement substitué, ou
les paires
($R_1$, $R_2$) et ($R_3$, $R_4$) représentent chacune, indépendamment l'une de l'autre, un radical alkylène contenant de 3 à 11 atomes de carbone.

2. Composés de formule I selon la revendication 1 dans lesquels $R_1$ représente un alkyle contenant de 1 à 5 atomes de carbone, $R_2$ l'hydrogène ou un alkyle contenant de 1 à 5 atomes de carbone, ou $R_1$ et $R_2$ forment ensemble un alkylène contenant de 4 à 7 atomes de carbone, $R_3$ représente un alkyle contenant de 1 à 7 atomes de carbone, un cyclo-alkyle contenant de 5 à 8 atomes de carbone, un phényle non substitué ou encore, dans le cas où $R_4$ représente H, un radical $-C(C_2H_5)=CH-CH_3$, et $R_4$ représente l'hydrogène ou un alkyle contenant de 1 à 5 atomes de carbone.

3. Composés de formule I selon la revendication 2 dans lesquels $R_3$ représente un alkyle contenant de 1 à 5 atomes de carbone, un phényle non substitué ou encore, dans le cas où $R_4$ représente l'hydrogène, un radical $-C(C_2H_5)=CH-CH_3$, et $R_4$ représente l'hydrogène ou un méthyle.

4. Composés de formule I selon la revendication 1 dans lesquels $R_1$ représente un méthyle ou un éthyle, $R_2$ l'hydrogène, un méthyle ou un éthyle, $R_3$ un alkyle contenant de 1 à 5 atomes de carbone ou un phényle non substitué, et $R_4$ l'hydrogène ou un méthyle.

5. Composé de formule I selon la revendication 1 dans lequel $R_1$ et $R_2$ représentent chacun un méthyle, $R_3$ représente un isopropyle et $R_4$ l'hydrogène.

6. Procédé de préparation de composés de formule I selon la revendication 1, procédé caractérisé en ce que:

– ou bien on réduit directement des composés de formule II:

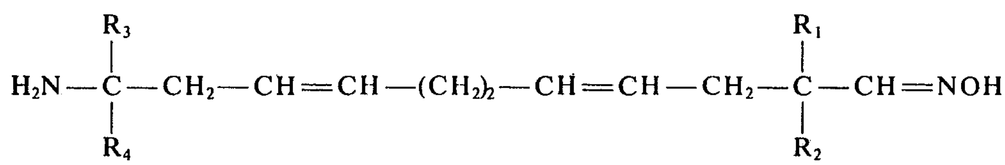

(II)

en composés de formule I,

– ou bien on déshydrate d'abord des composés de formule II en composés de formule III:

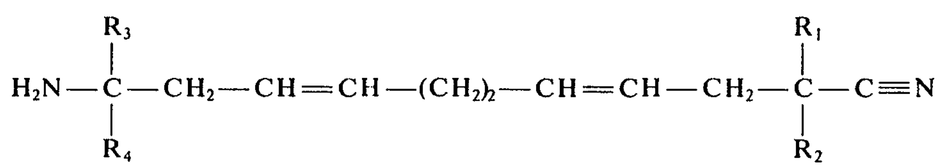

(III)

et ensuite on réduit les composés de formule III en composés de formule I,

les symboles $R_1$ à $R_4$ présents dans les formules précédentes ayant les significations qui leur ont été données à la revendication 1.